# EUROPÄISCHE PATENTSCHRIFT

(11) **EP 0 652 027 B1**
(45) Veröffentlichungstag und Bekanntmachung des Hinweises auf die Patenterteilung: **07.05.2003**
(21) Anmeldenummer: 94116303.2
(22) Anmeldetag: 17.10.1994
(51) Int. Cl.: A61M 25/06, A61M 5/158, A61M 5/32

(54) **Injektionsvorrichtung**
Injection device
Dispositif d'injection

(30) Priorität: 22.10.1993 CH 320093
(43) Veröffentlichungstag der Anmeldung: 10.05.1995
(73) Patentinhaber: F. HOFFMANN-LA ROCHE AG, 4002 Basel (CH)
(72) Erfinder: Cirelli, Giorgio, CH-4313 Möhlin (CH); Rothenhäusler, Benno, D-79541 Lörrach (DE); Steffen, Hans, CH-4410 Liestal (CH)
(74) Vertreter: Buntz, Gerhard

(56) Entgegenhaltungen:
- EP-A- 0 239 244
- EP-A- 0 272 530
- EP-A- 0 369 972
- DE-A- 4 200 595
- US-A- 4 170 993
- US-A- 4 632 671

## Beschreibung

Die Erfindung betrifft eine Vorrichtung zum Einbringen einer Wirkstofflösung in einen Patienten mit Mitteln zur Befestigung der Vorrichtung auf der Körperoberfläche und einer Kanüle zum Einstechen in die Haut oder in das darunter liegende Gewebe und zum Einleiten der Wirkstofflösung.

Im Rahmen der Vorbereitung eines Patienten auf einen Eingriff etc. wird heute üblicherweise ein venöser Zugang geschaffen, an den im Bedarfsfall Injektionsgeräte angeschlossen werden können. Im Bereich der Nachsorge, Therapie und Prävention ist diese Methode jedoch häufig mit Nachteilen verbunden, von denen die gravierendsten genannt seien. Das Setzen von Kanülen kann nur durch geschultes medizinisches Personal erfolgen. Patienten selbst sind in den meisten Fällen dazu nicht in der Lage, so dass Selbstmedikation bei Medikamenten, die injiziert werden, nicht möglich ist. Zum anderen entsteht durch das Setzen einer Kanüle insbesondere bei der Langzeittherapie eine Verletzung mit erhöhter Infektionsgefahr. Die Verletzungsgefahr bei der Handhabung bereits gebrauchter Kanülen stellt ein zusätzliches Risiko dar. Die genannten Nachteile führen dazu, dass aus Kosten- und Sicherheits-gründen häufig der subkutane oder intradermale Injektionsweg gewählt wird.

Für den Komfort des Patienten, speziell zur Erleichterung der Selbstmedikation wurden am Körper tragbare Geräte entwickelt, die eine Kombination aus Kanüle und Reservoir für die Wirkstofflösung darstellen. Beispielsweise ist aus EP - A - 272 530 ein tragbares Gerät zur subkutanen oder intradermalen Injektion einer flüssigen Wirkstofformulierung bekannt, das aus einem Vorratsbehälter, einer mit diesem in Verbindung zu bringenden Injektionsnadel, einer Pumpeinrichtung zum Entleeren des Behälters durch die Injektionsnadel und Befestigungsmitteln besteht, wobei die Nadel mittels einer Antriebsvorrichtung in die Haut des Patienten eingeschossen wird.

Vorrichtungen dieser Art, die ein Wirkstoffreservoir als integralen Bestandteil aufweisen, haben den Nachteil, dass für jede Wirkstoffformulierung ein separates Gerät erforderlich ist. Ausserdem verhindert die Begrenzung des Wirkstoffvolumens bei diesen Geräten deren Einsatz für eine Reihe von Anwendungen.

Eine andere Vorrichtung zum Einbringen eines Wirkstoffes in einen Patienten ist in US-A-4,632,671 beschrieben. Das in diesem Dokument gezeigte Gerät dient zum Füllen oder ggf. zum Entleeren eines im Patienten implantierten Reservoirs. Das für den Patienten unangenehme häufige Einstechen einer Kanüle wird dadurch vermieden, dass eine einmal gesetzte Kanüle über einen längeren Zeitraum an Ort und Stelle bleibt und mehrmals benutzt wird. Dies wäre für das Einbringen von Wirkstofflösung in das Hautoder Unterhautgewebe ungeeignet.

Der Erfindung liegt daher die Aufgabe zugrunde, ein Gerät bereitzustellen, das hohen Patientenkomfort und gleichzeitig grösstmögliche Flexibilität und Sicherheit bei intradermalen und subkutanen Injektionen bietet.

Erfindungsgemäss wird dies erreicht durch eine Vorrichtung gemäß Anspruch 1.

Im folgenden werden anhand der beiliegenden Zeichnungen Ausführungsbeispiele der Erfindung beschrieben.

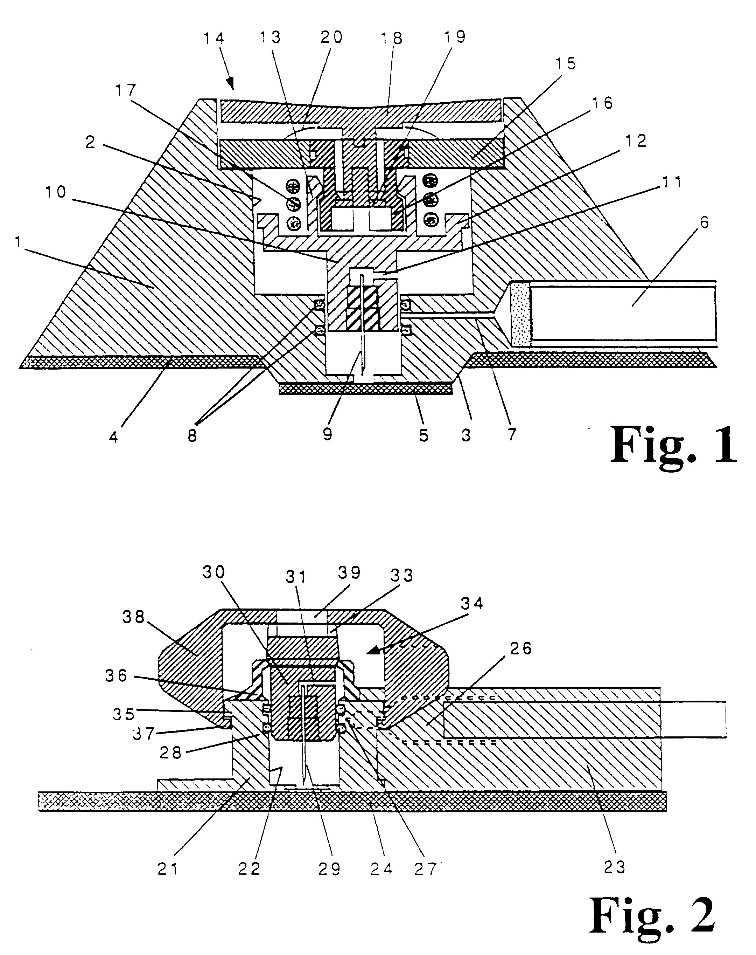

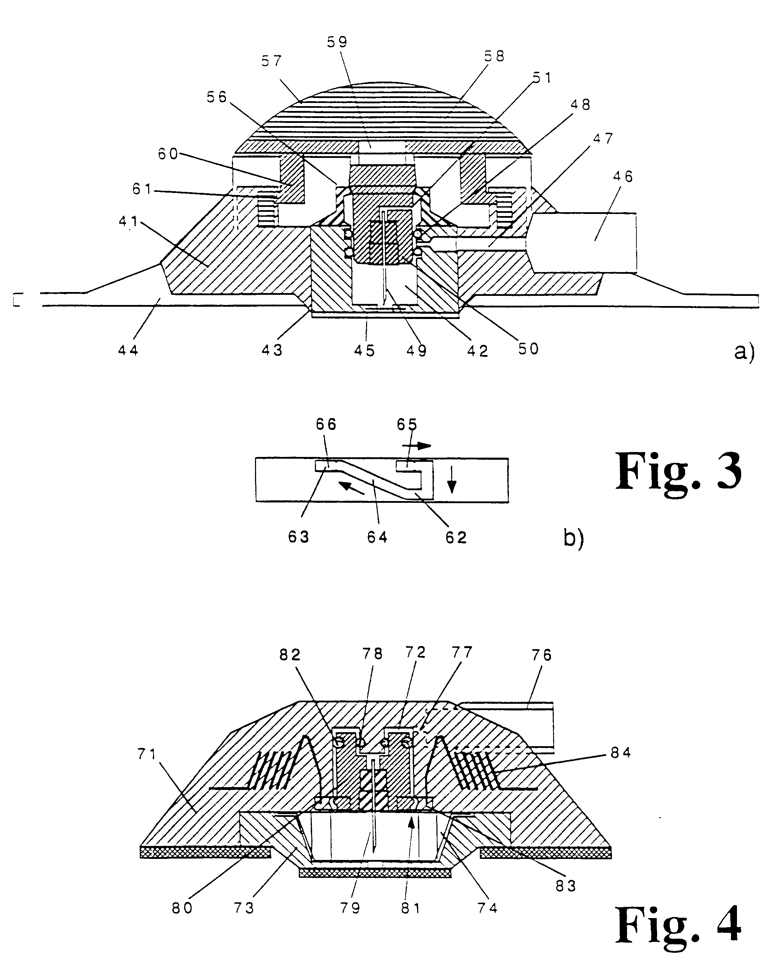

Die Fig. 2,3 zeigen unterschiedliche Ausführungsformen von Injektionseinrichtungen.

Die Figuren 1, 4 und 5 beinhalten Ausführungsformen, jeweils im Schnitt, die ausserhalb des Anspruchs umfanges liegen, aber zur Erleuterung der Erfindung dienen.

Die in Fig. 1 gezeigte Vorrichtung besitzt ein etwa kegelstumpfförmiges Gehäuse 1 mit einer konzentrischen Bohrung 2, die aus vier Bereichen mit von unten nach oben jeweils grösseren Durchmessern besteht. Die Grundfläche des Gehäuses weist einen konzentrischen Vorsprung 3 auf. Der den Vorsprung 3 umgebende Teil der Grundfläche ist mit einer Klebeschicht 4 zum Befestigen der Vorrichtung auf einer Körperstelle eines Patienten versehen, deren Material und Dicke vor allem im Hinblick auf den Tragekomfort und auf sicheres Halten der Vorrichtung ausgewählt sind. Unter diesem Gesichtspunkt wurde ein sog. Klebeschaum für die Schicht 4 ausgewählt. Die Stirnfläche des Vorsprunges 3 ist ebenfalls mit einer Klebeschicht 5 versehen, die vor allem dem zuverlässigen Fixieren der Haut dient. Zu diesem Zweck besteht die Klebeschicht 5 aus einem dünneren, verhältnismässig agressiv klebenden Material. Allerdings ist es durchaus möglich, dass die Klebeschichten 4 und 5 aus demselben Material bestehen.

Auf einer Stelle des Gehäuses ist ein Luer-Ansatz 6 angeordnet, der zur Verbindung der Vorrichtung mit einer Förderpumpe für eine Wirkstofflösung und über diese mit einem Reservoir dient. Vom Luer-Ansatz führt ein enger Kanal 7 zur zentralen Bohrung. Ueber und unter der Mündung des Kanals 7 in die Bohrung sind in der Innenwand der Bohrung O-Ringe 8 in entsprechende Ringnuten eingesetzt.

Als Kanüle 9 dient eine dünne Stahlkapillare von etwa 200 µ Durchmesser mit schräg geschliffenem Einstichende. Die Kanüle ist in einem Kanülenträger 10 gehalten. Der Kanülenträger 10 ist ein konzentrisch und axial verschiebbar in der Bohrung 2 angeordnetes Element, das in seiner oberen Endposition gezeigt ist. In seiner unteren Endposition liegt die untere Stirnfläche des Kanülenträgers auf dem Anschlag auf, der durch den Uebergang vom engsten Bereich der Bohrung 2 zu ihrem nächstgrösseren gebildet wird. Die Kanüle 9 erstreckt sich dann durch den als Nadeldurchlass dienenden engsten Bereich der Bohrung 2 und durch die Klebeschicht 5 hindurch, z.B. in das Gewebe der angrenzenden Haut eines Patienten.

Von der zylindrischen Seitenfläche des Kanülenträgers 10 führt ein sich im Inneren desselben erweiternder Kanal 11. In die Erweiterung des Kanals 11 mündet das der angeschliffenen Spitze der Kanüle 9 gegenüberliegende Ende derselben. In der unteren Position des Kanülenträgers befindet sich der Kanal 11 dem Kanal 7 im Gehäuse 1 gegenüber.

Der Kanülenträger besitzt einen Führungsflansch 12 dessen Durchmesser dem Innendurchmesser des dritten Bohrungsbereiches entspricht. Auf seiner oberen Fläche weist der Kanülenträger 10 eine hohlzylindrische Wand 13 auf, die auf ihrer Innenseite unmittelbar an ihrem oberen Ende mit einem umlaufenden Wulst versehen ist. Die Wand 13 mit dem Wulst wirkt mit dem nachfolgend zu beschreibenden Halte- und Auslösemechanismus 14 zusammen.

In den obersten Bereich der Bohrung 2 mit dem grössten Durchmesser ist ein Halte- und Auslösemechanismus 14 eingesetzt, der den Kanülenträger 10 im Ruhezustand in seiner oberen Endposition hält und durch Auslösen dessen Verschiebung in die untere Endposition ermöglicht. Zwischen dem Kanülenträger 10 und dem Halte- und Auslösemechanismus 14 befindet sich eine Schraubenfeder, die den Kanülenträger 10 nach unten drückt.

Der Halte- und Auslösemechanismus 14 besteht aus zwei axial gegeneinander verschiebbaren Teilen, von denen der untere aus einer ringförmigen Platte 15 und an deren zentraler Oeffnung nach unten ragenden, im wesentlichen eine hohlzylindrische Form bildenden Wandsegmenten 16 besteht. Die Platte 15 ist fest mit dem Gehäuse verbunden, d.h. verschraubt, verschweisst etc. Sie bildet das obere Widerlager der Feder 17 und muss daher relativ steif ausgebildet sein.

Die Wandsegmente 16 besitzen an ihren Aussenflächen einen konischen Absatz, der den Uebergang zwischen einem oberen kleineren zu einem unteren grösseren Achsabstand bildet. Auf diesem Absatz liegt der Wulst der Wand 13 auf, wodurch der Kanülenträger 10 in seiner oberen Endposition gehalten wird. Auf ihrer Innenseite besitzen die Wandsegmente 16 ebenfalls einen Absatz, in welchem ein nachfolgend zu beschreibender Spreizflansch 19 eingreift.

Der obere Teil des Halte- und Auslösemechanismus 14 besteht aus einem etwa pilzförmigen Auslösedrücker 18, an dessen unterem Ende sich der erwähnte Spreizflansch 19 befindet. Durch eine Tellerfeder 20 wird der Auslösedrücker in der gezeigten oberen Position gehalten. Der Spreizflansch 19 verhindert die Einwärtsbewegung der elastischen Wandsegmente 16 und damit die Auslösung der Abwärtsbewegung des Kanülenträgers 10.

Der Halte- und Auslösemechanismus 14 stellt in der vorliegenden Ausführungsform demnach einen Schnapper mit Verriegelung dar. Durch Entfernen der Verriegelung löst der Schnapper infolge des Druckes der Feder 17 selbsttätig aus.

Wenn nun die Vorrichtung an einer geeigneten Körperstelle eines Patienten angebracht, d.h. aufgeklebt, und mit einer Fördereinrichtung für eine Wirkstofflösung verbunden ist und wenn die Injektion ausgelöst werden soll, drückt der Patient den Drücker 18 gegen den Druck der Feder 20 nach unten. Dadurch wird der Spreizflansch 19 aus seinem Sitz im Inneren der Wandsegmente 16 herausgeschoben. Die Wandsegmente weichen dem von der Spiralfeder 17 über den Wulst auf den Absatz ausgeübten Druck nach innen aus, und der Kanülenträger 10 samt Kanüle 9 wird von der Feder 17 mit hoher Beschleunigung nach unten geschoben. Die Kanüle 9 wird durch die Klebeschicht 5 hindurch in das Patientengewebe geschossen. Durch die hohe Beschleunigung erfolgt das Eindringen der Nadel so schnell, dass der Patient keinen Schmerz spürt.

Die in Fig. 2 gezeigte Ausführungsform besitzt eine Reihe von gleichen Funktionselementen wie die gemäss Fig. 1. Einige Elemente haben lediglich eine andere Form, während anderen Elementen andere oder zusätzliche Funktionen zukommen.

Ein Gehäuse 21 besitzt eine Bohrung 22, die im vorliegenden Fall nur eine Abstufung ihres Durchmessers hat, nämlich die Verengung an ihrem unteren Ende zu einer Durchtrittsöffnung für eine Injektionskanüle 29. Das Gehäuse ist ebenfalls auf seiner Unterseite mit einer Klebeschicht 24 versehen, die hier durchgehend ausgebildet ist. In einer seitlich angesetzten Verlängerung 23 des Gehäuses ist ein Luer-Ansatz 26 angeordnet, der über einen Kanal 27 mit der Bohrung 22 verbunden ist. Ober- und unterhalb der Mündung des Kanals 27 in der Bohrung 22 liegen in entsprechenden Ringnuten zwei O-Ringe 28.

Die Kanüle 29 sitzt auch hier in einem Kanülenträger 30, der im wesentlichen zylindrisch ausgebildet ist und einen Kanal 31 zur Verbindung der Kanülenrückseite mit der Bohrung 22 aufweist. Seine Mantelfläche besitzt einen durch eine Querschnittserweiterung nach oben gebildeten konischen Absatz. Der Kanülenträger 30 ist über ein ringförmiges glasklares Sichtelement 33 fest mit einer den gesamten Mechanismus umgreifenden Kappe 38 verbunden, die aus der in der Figur gezeigten oberen Position nach unten bewegbar ist und als Auslösekopf dient. Die Kappe 38 besitzt in ihrem Zentrum ein Sichtfenster 39.

Auf der Gehäuseoberfläche befinden sich um den Rand der Bohrung 22 herum angeordnete Wandsegmente 36, die an ihrem oberen Rand mit zur Achse gerichteten Vorsprüngen versehen sind, die unter den konischen Absatz des Kanülenträgers 30 greifen. Die Wandsegmente 36 sind elastisch und bilden mit dem konischen Absatz des Kanülenträgers zusammen einen Halte- und Auslösemechanismus 34. Durch Druck mit dem Finger auf die Kappe 38 werden sie nach aussen gebogen, bis sie bei ausreichendem Druck den Kanülenträger 30 freigeben. Durch den zur Ueberwindung der Festhaltekraft erforderlichen Fingerdruck wird beim Freigeben des Kanülenträgers 30 die Nadel in die Haut des Patienten gedrückt. Das obere Ende des Kanülenträgers 30 ist konisch verjüngt und so ausgebildet, dass die elastischen Wandelemente 36 einen nach unten gerichteten Druck auf den Kanülenträger ausüben, welcher die Nadel nach dem Eindrücken in der unteren Einstichposition hält.

Die äussere Oberfläche der Kappe 38 ist so geformt, dass sie mit den Fingern gegriffen und wieder in ihre Ausgangsstellung zurückgezogen werden kann. An ihrem unteren Rand ist die Kappe mit einem nach innen gerichteten Vorsprung 37 versehen, welcher zusammen mit einem entsprechenden Absatz 35 des Gehäuses ein Abziehen der Kappe über die Ausgangsstellung hinaus verhindert. Dadurch wird erreicht, dass die Kanüle nach Beendigung der Infusion wieder vollständig in das Gehäuse zurückgezogen werden kann.

In der unteren Endposition von Kappe 38 und Kanülenträger 30 befinden sich die Vorsprünge der Wandsegmente 36 auf der Höhe des Sichtelementes 33 und liegen an ihm an. Die Wandsegmente 36 sind farbig hervorgehoben, so dass sie in dieser Position durch das Sichtfenster 39 gut sichtbar werden und dadurch anzeigen, dass die Kanüle 29 eingestochen ist. Eine solche Sichtkontrolle ist oft von Vorteil, weil der Patient den Einstich nicht spürt.

Fig. 3a zeigt eine Vorrichtung, bei der das Gehäuse 41 sehr ähnlich ausgebildet ist, wie in Fig. 1 mit Bohrung 42, Vorsprung 43, Klebeschichten 44 und 45, sowie mit Luer-Ansatz 46, Kanal 47 und O-Ringen 48 in der Wand der Bohrung 42. Der Kanülenträger 50 mit Kanüle 49 und Verbindungskanal 51 und die ihn haltenden Wandsegmente 56 sind gleich wie bei der Vorrichtung gemäss Fig. 2. Die Kappe 57, die auch hier ein Sichtfenster 59 besitzt, ist mit einem Knebel 58 versehen, durch den sie gedreht werden kann. Sie besitzt zudem mehrere von ihrer Unterseite nach unten ragende Finger 60, die wiederum mit radial nach aussen gerichteten Nocken 61 versehen sind. Diese Nocken 61 gleiten in Nuten, die in der Wand des oberen Bohrungsbereiches vorgesehen sind und von denen eine in Fig. 3b gezeigt ist. Die Nut weist einen senkrecht nach unten verlaufenden Schenkel 62 und einen rampenförmigen schrägen Schenkel 63 auf. Die beiden Schenkel sind unten durch ein kurzes Horizontalstück 64 verbunden. An ihren oberen Enden ist jeweils ein horizontales Nutstück 65,66 angeschlossen. Das in der Zeichnung rechte horizontale Nutstück 65, das sich oben am senkrechten Schenkel 62 befindet definiert die Ruheposition vor dem Gebrauch der Vorrichtung. Das andere horizontale Nutstück 66 oben am schrägen Schenkel 63 definiert die Position nach dem Gebrauch der Vorrichtung. In diese Position werden Kanülenträger 50 und Injektionskanüle 49 durch Drehen des Knebels 58 zurückgezogen bevor die Vorrichtung vom Patienten abgenommen wird. Dieser Rückzug der Kanüle verhindert sowohl eine mögliche Verletzung des Patienten durch Verkippen der Kanüle beim Abnehmen, als auch Verletzungen und damit Infektionen der medizinischen Helfer durch die vorstehende Kanüle.

Die in Figur 4 gezeigte Vorrichtung besitzt keine vom Patienten manuell zu betätigende Auslösung des Kanülenvorschubs. Das sehr flache, nur ca. 10 mm hohe Gehäuse 71 ist nach oben geschlossen und besitzt eine nach unten offene konzentrische Kammer 72. Es ist ausserdem zweiteilig und besitzt einen in der Grundfläche eingesetzten Teil 73, der gleichzeitig einen die Durchtrittsöffnung umgebenden vorspringenden Teil der Grundfläche bildet. Zwischen dem Hauptteil des Gehäuses 71 und dem eingesetzten Teil 73 ist eine die zwischen den beiden Teilen gebildete Kammer umschliessende Schutzfolie 74 angeordnet. Im oberen Teil des Gehäuses ist ein Luer-Ansatz 76 angeordnet, der durch einen Kanal 77 mit der Kammer 72 verbunden ist.

An einem von oben in die Kammer hineinragenden konzentrischen Vorsprung ist in eine entsprechende umlaufende Nut ein O-Ring 78 eingelegt.

Eine Kanüle 79 sitzt in einem Kanülenträger 80, der im wesentlichen der Form der Kammer 72 angepasst ist. An seinem unteren Rand besitzt der Kanülenträger 80 einen umlaufenden, in eine entsprechende Ringnut des Gehäuses eingreifenden Vorsprung, der zusammen mit der Nut einen Schnapper 81 bildet. Dieser hält im Ruhezustand den Kanülenträger in der gezeigten Position fest.

Nahe dem oberen Rand seiner zylindrischen Aussenfläche besitzt der Kanülenträger eine umlaufende Ringnut, in die ein O-Ring 82 eingesetzt ist. Durch die beiden O-Ringe 78 und 82 entsteht zwischen dem Kanülenträger und der oberen Stirnfläche der Kammer ein ringförmiger abgeschlossener Raum, der nur durch den Kanal 77 mit dem Luer-Ansatz 76 verbunden ist.

An seinem unteren Rand besitzt der Kanülenträger ferner zwei radial nach aussen ragende Stifte 83. An diesen greifen die Endstücke zweier Schenkelfedern 84 an. Diese sind so vorgespannt, dass sie den Kanülenträger nach unten befördern, sobald er freigegeben ist.

Die Funktion dieser Ausführungsform ist wie folgt:

Wenn die Vorrichtung an einer Körperstelle des Patienten befestigt ist, wird über den Luer-Ansatz die Verbindung zur Fördereinrichtung für die Wirkstofflösung (nicht gezeigt) hergestellt. Die Förderpumpe erzeugt einen Druck im oberen Bereich der Kammer 72. Die dadurch auf den Kanülenträger 80 wirkende Kraft addiert sich zur Kraft der Federn 84. Sobald die addierte Kraft die Haltekraft des Schnappers 81 überschreitet, bewegt sich der Kanülenträger nach unten. Die Federn 84 übernehmen nun den Vortrieb des Kanülenträgers. Nach einer ersten kurzen Wegstrecke gelangt die innere obere Kante des Kanülenträgers unter den O-Ring 78 und gibt somit den Weg für die Wirkstofflösung frei zum oberen Ende der Kanüle 79. Die Wirkstofflösung kann durch die Kanüle 79 in die Haut des Patienten gelangen.

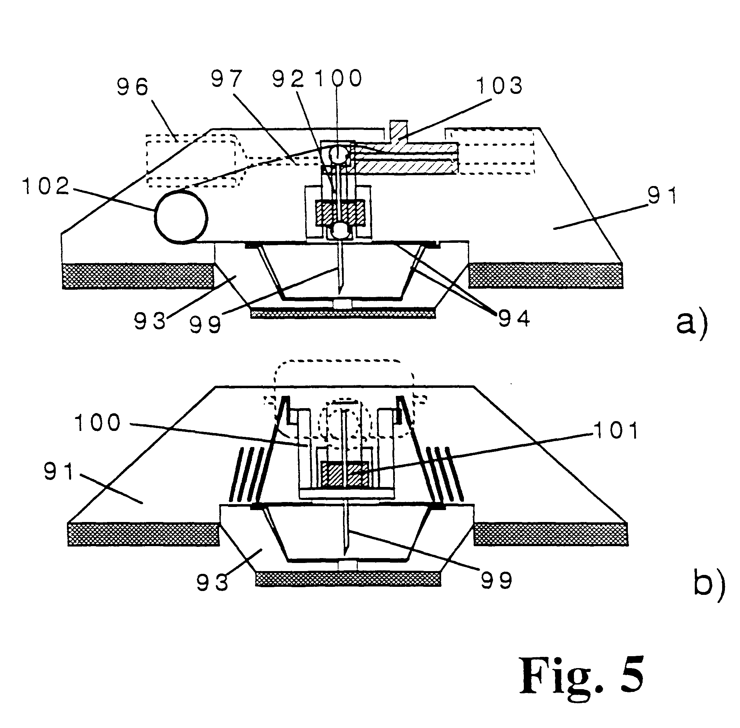

Die in Figur 5 gezeigte Vorrichtung ist in zwei zueinander senkrechten Querschnitten dargestellt, um die Einzelheiten deutlicher zu machen. Bei dieser Vorrichtung ging es darum, die Bauhöhe noch weiter zu verkleinern. Das Gehäuse 91 ist sehr flach, d.h. es hat eine Höhe von weniger als 10 mm. Eine im Gehäuse zentral angeordnete Kammer 92 ist wiederum von unten offen und nach oben geschlossen. Für den Zusammenbau ist das Gehäuse zweiteilig. Der untere Teil 93 bildet einen zentralen, konzentrischen Vorsprung, der über die Grundfläche des Gehäuses hinausragt. Zwischen dem unteren Teil 93 und dem Hauptteil des Gehäuses 91 sind Schutzfolien 94 angeordnet, welche den zwischen den beiden Gehäuseteilen entstehenden Hohlraum abdichten. Im oberen Gehäuseteil 91 ist wieder ein Luer-Ansatz 96 angeordnet, der durch einen Kanal 97 mit der Kammer 92 verbunden ist.

Eine Kanüle 99 befindet sich in einem Kanülenträger 100 eingesetzt und bewegt sich ansonsten aber frei in einer im unteren Teil der Kammer befindlichen elastischen Dichtung 101.

Der Kanülenträger 100 besteht im wesentlichen nur aus einem U-förmigen Element, an dessen oberen Schenkelenden sich Ansatzpunkte für die Kraftübertragung von Schenkelfedem 102 befinden. Die Federn 102 sind so vorgespannt, dass sie den Kanülenträger mit der Kanüle 99 nach unten treiben, sobald diese freigegeben sind.

Der Kanülenträger 100 wird in der in Figur 5 gezeigten oberen Ruheposition durch einen Schieber 103 festgehalten. Wird der Schieber 103 nach rechts geschoben, so gibt er den Kanülenträger 100 frei, und die Feder 102 kann den Kanülenträger 100 samt Kanüle 99 nach unten bewegen und somit die Kanüle 99 in die Haut des Patienten einstechen.

## Patentansprüche

1. Vorrichtung zum Einbringen einer Wirkstofflösung in einen Patienten mit einem Gehäuse (21,41); Mitteln (24,44,45) zur Befestigung des Gehäuses (21,41) auf der Körperoberfläche des Patienten; einer Kanüle (29,49) zum Einleiten der Wirkstofflösung; einer selbsttätigen Vorschubeinrichtung mit einem Halte- und Auslösemechanismus (34,57,58) zum Einschiessen der Kanüle (29,49) in die Haut; einen nur im eingestochenen Zustand der Kanüle offenen Verbindungskanal (27,31,47,51) zwischen einem Anschlusselement (26,46) zur Verbindung mit einer Fördereinrichtung für die Wirkstofflösung, und der Kanüle (29,49),
**dadurch gekennzeichnet, dass**
- das Anschlusselement (26,46) an der Aussenseite des Gehäuses (21,41) angeordnet ist, und dass
- Mittel (38,58) zum Rückzug des Kanülenträgers (30,50) und der Kanüle (29,49) vorgesehen sind.

2. Vorrichtung nach Anspruch 1, **dadurch gekennzeichnet, dass** der Halte- und Auslösemechanismus (34,57,58) einen Schnapper (36,56) besitzt.

3. Vorrichtung nach Anspruch 1 oder 2, **dadurch gekennzeichnet, dass** der Halte und Auslösemechanismus (34,57,58) einen Auslöseknopf (38,58) mit einem Sichtfenster (39,59) zur Kontrolle der Position des Kanülenträgers (30,50) und der Kanüle (29,49) besitzt.

4. Vorrichtung nach einem der vorherigen Ansprüchen 1 zu 3, **dadurch gekennzeichnet, dass** der Kanülenträger (50) mit seitlichen, in einer Nut (62,63,64,65,66) des Gehäuses (41) geführten Nocken (61) versehen ist.

## Claims

1. A device for the introduction of an active substance solution into a patient with a housing (21, 41); means (24, 44, 45) for fastening the housing (21, 41) to the surface of the body of the patient; a cannula (29, 49) for the introduction of the active substance solution; an automatic advancer unit with a retaining and releasing mechanism (34, 57, 58) for propelling the cannula (29, 49) into the skin; a connecting channel (27, 31, 47, 51), which is open only in the inserted state of the cannula, between a connecting element (26, 46) for connection to a conveying unit for the active substance solution and the cannula (29, 49)
**characterized in that**
- the connecting element (26, 46) is arranged on the outside of the housing (21, 41), and **in that**
- means (38, 58) are provided for withdrawal of the cannula holder (30, 50) and the cannula (29, 49).

2. A device according to claim 1, **characterized in that** the retaining and releasing mechanism (34, 57, 58) has a spring-loaded catch (36, 56).

3. A device according to claim 1 or 2, **characterized in that** the retaining and releasing mechanism (34, 57, 58) has a releasing button (38, 58) with a viewing window (39, 59) to check the position of the cannula holder (30, 50) and the cannula (29, 49).

4. A device according to any one of foregoing claims 1 to 3, **characterized in that** the cannula holder (50) is provided with toes (61) on the side which are guided in a groove (62, 63, 64, 65, 66) of the housing (41).

## Revendications

1. Dispositif d'injection d'une solution de substance active dans un patient, comprenant un boîtier (21, 41), des moyens (24, 44, 45) servant à fixer le boîtier (21, 41) sur le corps du patient, une canule (29, 49) servant à injecter la solution de substance active, un dispositif d'avance automatique doté d'un mécanisme de retenue et de déclenchement (34, 57, 58) servant à introduire la canule (29, 49) dans la peau, un canal de communication (27, 31, 47, 51) ouvert seulement lorsque la canule est introduite, lequel canal de communication est disposé entre un élément de raccordement (26, 46) servant à la jonction avec un dispositif de transport pour la solution de substance active, et la canule (29, 49),
**caractérisé**
- **en ce que** l'élément de raccordement (26, 46) est disposé sur le côté extérieur du boîtier (21, 41), et
- **en ce que** des moyens (38, 58) sont prévus pour le retrait du porte-canule (30, 50) et de la canule (29, 49).

2. Dispositif selon la revendication 1, **caractérisé en ce que** le mécanisme de retenue et de déclenchement (34, 57, 58) comprend un élément à déclic (36, 56).

3. Dispositif selon la revendication 1 ou 2, **caractérisé en ce que** le mécanisme de retenue et de déclenchement (34, 57, 58) comprend un bouton de déclenchement (38, 58) doté d'une fenêtre de visualisation (39, 59) servant au contrôle de la position du porte-canule (30, 50) et de la canule (29, 49).

4. Dispositif selon l'une des revendications précédentes 1 à 3, **caractérisé en ce que** le porte-canule (50) est doté de cames latérales (61) guidées dans une rainure (62, 63, 64, 65, 66) du boîtier (41).
